# EUROPÄISCHE PATENTANMELDUNG

(11) **EP 4 339 276 A2**
(43) Veröffentlichungstag der Anmeldung: **20.03.2024**
(21) Anmeldenummer: 23197820.6
(22) Anmeldetag: 17.09.2023
(51) Int. Cl.: C12M 1/32, C12M 3/06, C12M 1/00

(54) **GRUNDKÖRPER EINES MEHRKAMMER-BIOCHIPS, HERSTELLUNG DES MEHRKAMMER-BIOCHIPS UND DESSEN VERWENDUNG FÜR DIE ETABLIERUNG VON ORGAN- UND KRANKHEITSMODELLEN UND SUBSTANZTESTUNGEN**

(30) Priorität: 18.09.2022 DE 102022123877
(71) Anmelder: Dynamic42 GmbH, 07745 Jena (DE)
(72) Erfinder: Abdo, Nader, 07743 Jena (DE); Rennert, Knut, 07743 Jena (DE); Raasch, Martin, 99423 Weimar (DE)
(74) Vertreter: Carlsohn, Marc René

(57) **Zusammenfassung**

Die Erfindung betrifft einen Grundkörper (1) eines Mehrkammer-Biochips (2), aufweisend einen Boden (3) mit einem darauf befindlichen und auf seiner dem Boden (3) abgewandten Seite offenen Rahmen (4), wobei durch den Rahmen (4) ein Innenraum (6) umgrenzt ist. Weiterhin ist wenigstens ein erster Steg (5), vorzugsweise sind aber ein erster Steg (5) und ein zweiter Steg (7), vorhanden. Durch die Stege (5, 7) und den Rahmen (4) ausgebildete und mittels Membranen (11, 12, 13) voneinander abgegrenzte Kulturkammern (8, 9, 10) sind mittels Kanälen (14) kontaktiert und dienen der Erzeugung biologischer Modellsysteme.

Der Grundkörper (1) ist insbesondere einstückig (monolithisch) ausgebildet. Die Erfindung betrifft zudem einen Mehrkammer-Biochip (2), ein Set und ein Verfahren zur Herstellung eines Mehrkammer-Biochips (2) sowie zu Verwendungen desselben.

## Beschreibung

Die Erfindung betrifft einen Grundkörper, mittels dem ein Mehrkammer-Biochip für eine Reihe innovativer Anwendungen hergestellt werden kann.

Biochips im Sinne dieser Anmeldung dienen der Nachbildung biologischer Systeme, wie z.B. Organen oder Geweben in einem experimentellen Setup, indem in Reaktionsräumen biologische Strukturen und Situationen/Umgebungsbedingungen künstlich, aber möglichst realitätsnah nachempfunden werden. Beispielsweise kann ein Biochip aus einer Anzahl übereinander angeordneter Komponenten gebildet sein, durch deren Zusammenwirken mindestens eine Kulturkammer, oft aber zwei Kulturkammern gebildet sind. Diese können voneinander mittels Membranen ausgewählter Eigenschaften abgetrennt sein, wobei die Kulturkammern mit einem Medium oder mehreren Medien befüllt werden. Optional können in die Medien verschiedene Substanzen wie beispielsweise Nährstoffe, zu untersuchende Wirkstoffe, synthetische Materialien als auch Aerosole, Zellen, Mikroorganismen und/oder Sphäroide eingebracht sein, um gezielt biologische Systeme oder spezifische Situationen/Umgebungen unter kontrollierten Bedingungen nachzubilden.

Eine Möglichkeit der Ausführung solcher Biochips ist beispielsweise aus der Publikation von Raasch, M. et al. (Raasch, M. et al., 2016; An integrative microfluidically supported in vitro model of an endothelial barrier combined with cortical spheroids simulates effects of neuroinflammation in neocortex development; Biomicrofluidics 10; doi 10.1063/1.4955184) bekannt. Zwei Grundelemente, die jeweils eine vorläufige Kulturkammer ausbilden, werden jeweils mit einer Trennmembran kombiniert und anschließend per Klebung zusammengefügt und mit Abschlussfolien und zum Teil mit Klebstoffen oder verbindenden Stoffen abgedichtet. Ein Befüllen der entstehenden drei übereinander angeordneten und durch die beiden Trennmembranen getrennten Kulturkammern erfolgt über ebenfalls in den beiden Grundelementen ausgeformte Kanäle. Auf diese Weise können mindestens zwei Kulturkammern auf kleinem Raum geschaffen und unter Laborbedingungen, also unter Kontrolle beispielsweise der verwendeten biologischen Strukturen sowie der Zusammensetzung, Temperatur und gegebenenfalls Strömung der Medien, betrieben und untersucht werden. Die Verwendung solcher gattungsgemäßen Biochips in einem Zellkulturinkubator ermöglicht zudem einen Betrieb in einer beispielsweise hinsichtlich Temperatur und Zusammensetzung kontrollierten umgebenden Atmosphäre.

Eine Detektion der Vorgänge im Inneren der Kulturkammern kann beispielsweise mittels einer optischen Analyse durch die transparenten Fenster des Biochips hindurch erfolgen (z.B. per Durchlichtmikroskopie-Analysen, Live Cell Imaging). Ferner können über die Kanäle Substanzen eingebracht werden, um die Barriere/Dichtigkeit/Durchlässigkeit der biologischen Strukturen über die Trennmembranen hinweg oder Transportprozesse an den biologischen Strukturen zu begutachten und/oder die Reaktionen von Proteinen auf den Oberflächen und/oder im Inneren der biologischen Strukturen zu analysieren. Weiterhin können biologische Strukturen und eingebrachte Stoffe wie Medien (auch angereichert mit den oben genannten Substanzen) für optische, molekularbiologische und chemische Analysen entnommen werden.

Ein vorstehend beschriebener Biochip muss jedoch aus einer Anzahl unterschiedlicher Elemente schichtweise aufgebaut werden, wobei jede Schicht einerseits dicht mit den jeweils benachbarten Elementen zu verbinden ist und andererseits die Anforderungen an die gewünschten Dimensionen der Kulturkammern und die dadurch bestimmten strömungstechnischen und physiologischen Rahmenbedingungen sicher eingehalten werden müssen. Das heißt auch, dass insbesondere die Grundelemente mit einer hohen Präzision und Maßhaltigkeit hergestellt werden müssen, wodurch die Herstellungskosten hoch sind.

Bei Experimenten der Erfinder zeigte sich ferner, dass durch eine Verklebung der Grundelemente Spannungen auftreten, die zu einer Verformung des Biochips führen und die Klebung Alterungsprozessen unterliegt, wodurch der Biochip undicht wird und seine Funktion verliert. Außerdem muss die Trennmembran nachteilig bereits vor dem Verkleben der Grundelemente eingebracht werden. Dies ist mit dem Risiko einer Kontamination der Membran durch z.B. Klebstoff verbunden.

Der Erfindung liegt die Aufgabe zu Grunde, eine Möglichkeit vorzuschlagen, mittels der die aus dem Stand der Technik bekannten Nachteile verringert bzw. gelöst und ein Mehrkammer-Biochip zur Verfügung gestellt werden kann, der für eine Vielzahl experimenteller Anwendungen genutzt werden kann.

Die Aufgabe ist durch die Gegenstände der unabhängigen sowie der nebengeordneten Ansprüche gelöst. Vorteilhafte Weiterbildungen sind Gegenstand der abhängigen Ansprüche.

Gelöst wird die Aufgabe der Erfindung mittels eines Grundkörpers eines Mehrkammer-Biochips, der einen Boden mit mindestens einem darauf befindlichen und mindestens auf seiner dem Boden abgewandten Seite offenen Rahmen aufweist. Dabei ist durch den Rahmen ein Innenraum umgrenzt. Der Rahmen kann als ein den Innenraum umlaufender Steg ausgebildet sein. Er kann in einer vorteilhaften, weil leichter herzustellenden Möglichkeit auch durch eine flächige, insbesondere rechteckige, Ausprägung des Grundkörpers gegeben sein, die eine entsprechend große, insbesondere der Höhe des Innenraums entsprechende, Materialstärke aufweist. Auf den Boden können mehrere Rahmen aufsetzen, die mehrere Innenräume abgrenzen. Alternativ können bei der Variante der flächigen Ausprägung des Grundkörpers auch mehrere Innenräume von der flächigen Ausprägung umgrenzt sein.

Es ist ferner mindestens ein innerhalb jedes Innenraums auf dem Boden aufsetzender und um eine erste Fläche umlaufender erster Steg ausgebildet, dessen Höhe geringer als die Höhe des Rahmens ist. Der erste Steg kann dabei freistehend oder als eine Stufe in dem Rahmen ausgebildet sein. Der Steg weist eine dem Innenraum zugewandte erste Seitenfläche und dem Boden gegenüberliegend eine erste Auflagefläche auf. Die erste Auflagefläche ist zur Auflage einer ersten Trennmembran ausgebildet. Durch die erste Seitenfläche des ersten Stegs und die von dem ersten Steg umfangene Fläche ist eine untere vorläufige Kulturkammer (erste Kammer) umgrenzt. Der verbleibende Innenraum zwischen der ersten Auflagefläche und der Oberseite des Rahmens bildet eine obere vorläufige Kulturkammer (zweite Kammer). Die von dem ersten Steg umfangene Fläche kann vieleckig, bevorzugt viereckig, insbesondere rechteckig, aber auch rund ausgebildet sein. Vorteilhaft läuft die Auflagefläche des Stegs in einer bezüglich des Bodens einheitlichen Höhe um die umfangene Fläche. Die untere vorläufige Kulturkammer sowie die obere vorläufige Kulturkammer sind jeweils durch mindestens je einen separaten, an einer Außenseite des Grundkörpers mündenden Kanal mit der Umgebung verbunden.

In weiteren Ausführungen des erfindungsgemäßen Grundkörpers ist die untere vorläufige Kulturkammer und/oder die obere vorläufige Kulturkammer mit mindestens zwei separaten Kanälen mit der Umgebung verbunden. Die Kanäle erlauben im montierten Zustand des Mehrkammer-Biochips vorteilhaft die Zuleitung und/oder die Ableitung von Medien in beziehungsweise aus den Kulturkammern.

Um beispielsweise die Kultivierung von Sphäroiden von etwa 1 mm Größe zu erlauben, sind wenigstens eine Kulturkammer und die zugehörigen Kanäle mit einer lichten Höhe beziehungsweise einem Durchmesser von mehr als 1 mm, beispielsweise von 1,1 oder 1,2 mm, konstruktiv ausgelegt.

Der erfindungsgemäße Grundkörper ist aus einem einzigen Werkstück gebildet (monolithisch) und hat bevorzugt Objektträgerformat (76 mm x 26 mm ± 3 mm). Die monolithische Ausführung erlaubt eine einfachere Handhabung gegenüber Lösungen gemäß dem Stand der Technik, da nicht zwei oder mehr Grundelemente miteinander zu verbinden sind, deren Durchführungen (Kanäle, Innenräume, Kulturkammern o.ä.) vor dem Verbinden justiert und verklebt werden müssen. Außerdem ist der Grundkörper in einer monolithischen Bauweise stabiler, er behält seine ursprüngliche Form bei (kein spannungsbedingtes Verbiegen des Körpers), er wird nicht undicht an Klebstellen und ist somit praxistauglicher in der Handhabung und Nutzung. Insbesondere können höhere Flussraten der zugeführten Medien (Perfusionsgeschwindigkeiten) erreicht werden, ohne dass es zu Undichtigkeiten kommt. Dies ist insbesondere bei der Etablierung von Organmodellen vorteilhaft, weil dort über möglichst lange Zeiträume Perfusionsgeschwindigkeiten wie in vivo aufrechterhalten werden müssen. Der erreichte Verzicht auf Klebstellen hat zudem vorteilhaft zur Folge, dass die im Stand der Technik zwingend notwendigen Klebstoffe entfallen können, die mit den biologischen Strukturen nachteilig interagieren konnten. Außerdem können die Anforderungen an die Maßhaltigkeit des erfindungsgemäßen Grundkörpers geringer ausfallen als die an einen aus mehreren Elementen zusammengesetzten Biochip.

In einer weiteren vorteilhaften Ausführung des Grundkörpers ist ein innerhalb des Innenraums auf dem Boden aufsetzender und um eine zweite Fläche umlaufender zweiter Steg ausgebildet. Dessen Höhe ist geringer als die Höhe des Rahmens, jedoch größer als die Höhe des ersten Stegs. Auch der zweite Steg kann freistehend oder als eine Stufe in dem Rahmen ausgebildet sein und weist eine dem Boden gegenüberliegende Auflagefläche, nachfolgend als zweite Auflagefläche bezeichnet, auf. Die zweite Auflagefläche ist zur Auflage einer zweiten Trennmembran ausgebildet. Zwischen der ersten Auflagefläche und der zweiten Auflagefläche bildet der zweite Steg eine dem Innenraum zugewandte zweite Seitenfläche aus. Durch die von dem zweiten Steg umfangene zweite Fläche und die zweite Seitenfläche ist eine mittlere vorläufige Kulturkammer (dritte Kammer) umgrenzt. Die von dem zweiten Steg umfangene Fläche kann vieleckig, bevorzugt viereckig, insbesondere rechteckig, aber auch rund ausgebildet sein. Bevorzugt entspricht die geometrische Form der von dem zweiten Steg umfangenen Fläche derjenigen der von dem ersten Steg umfangenen Fläche. Auch die mittlere vorläufige Kulturkammer ist mit mindestens einem Kanal mit einer Außenseite des Grundkörpers verbunden.

Wie weiter unten noch erläutert werden wird, kann mittels einer derartigen Ausführung des Grundkörpers in vorteilhafter Weise ein Mehrkammer-Biochip mit drei oder mehr übereinander angeordneten Kulturkammern hergestellt werden.

Vorteilhaft umfasst das Material, aus dem der Grundkörper hergestellt ist, einen spritzgussfähigen, biokompatiblen Kunststoff. Beispiele solcher Kunststoffe sind Polyester wie Polyurethane (PU), Polyimide, Styrene (SEBS), Polypropylene (PP), Polystyrene (PS), Polycarbonat (PC), Polyethylenterephthalat (PET) und zyklische Polyolefine (COP und COC). Der Materialwahl entsprechend erfolgt die Herstellung des Grundkörpers einstückig in einem Spritzguss- oder Gießverfahren unter Bereitstellung der entsprechenden Spritzguss- oder Gießwerkzeuge.

Im Stand der Technik werden Biochips oft auch aus Polydimethylsiloxanen (PDMS) hergestellt. Die Anwendung PDMS-basierter Chips für komplexe Zell-, Organ- und Krankheitsmodelle gestaltet sich jedoch aufwändig, da das PDMS im Gegensatz zu den vorgenannten Kunststoffen aktiviert werden muss, um für biologische Strukturen wie z.B. Zellkulturen geeignet zu sein. Um die mittels eines Biochips zu untersuchenden Systeme aus biologischen Strukturen und Medien so wenig wie möglich zu beeinflussen, sollten die Materialien des Biochips im Idealfall inert sein. Allerdings ist beispielsweise von dem häufig verwendeten Material PDMS bekannt, dass dieses gegenüber einigen chemischen Verbindungen hohe Bindekapazitäten aufweist (Auner et al., (2019): Chemical-PDMSbinding kinetics and implications for bioavailability in microfluidic devices; Lab Chip 19: 864-874). Derartige Bindekapazitäten können einen schwer abzuschätzenden Einfluss auf Untersuchungen (z.B. Substanztestungen) haben, die mit aus PDMS bestehenden Biochips durchgeführt werden. So adsorbieren z. B. Substanzen mit einem logP größer 1,8 und einem niedrigen Hydrogen donor count stark an PDMS, was eine Wirkstofftestung und die Interpretation der erhaltenen Daten erheblich erschwert. Beispielsweise besitzt die Substanz Propiconazol einen logP von 3,72 und einen sogenannten Hydrogen donor count von 0. Es ist sehr hydrophob und kann in PDMS-Chips nach 24 Stunden nur noch mit weniger als 30% der Ausgangskonzentration im Kulturmedium nachgewiesen werden, da es irreversibel an PDMS bindet (Auner et al. 2019). Dieses Problem betrifft vor allem, aber nicht ausschließlich, die klassische Wirkstoffgruppe der *small molecules.* Viele pharmazeutische Wirkstoffe gehören zu dieser Wirkstoffgruppe, wodurch PDMS-basierte Biochips als Testsysteme nicht geeignet sind. Zudem ist ein einteiliger Grundkörper für einen Mehrkammer-Biochip aus nur einem einzigen Werkstück ferner mittels PDMS nicht herstellbar, da bei diesem Werkstoff die notwendige Trennmembran nur zwischen zwei Einzel-Bauteilen fixiert werden kann.

In einer vorteilhaften Ausführung des erfindungsgemäßen Grundkörpers ist dieser daher aus Polybutylenterephthalat (PBT) gefertigt.

PBT ist ein Polymer, das üblicherweise zur Herstellung von Produkten verwendet wird, die einer hohen mechanischen Belastung unterliegen und/oder die wiederholt mit heißen Medien in Kontakt gelangen. Typische Verwendungen von PBT sind beispielsweise Gleitlager, Ventilteile, Schrauben, Teile für Haushaltsgeräte wie Kaffeemaschinen oder Haartrockner und Teile für Medizinprodukte wie Anschlüsse für Pulsoximeter, Spitzen für elektrochirurgische Instrumente und Clips für Atemmasken. Die Herstellung von medizinprodukte-geeigneten PBT-Ausgangspolymeren kann sogar GMP-konform realisiert werden. PBT ist für die Spritzguss-Verarbeitung aufgrund des günstigen Abkühlungs- und Prozessverhaltens sehr gut geeignet.

Dieses für die Verwendung in Zellkulturkammern ungewöhnliche Material zeigte in Versuchen sehr geringe Bindekapazitäten gegenüber einer Reihe von Bestandteilen der verwendeten Medien, so dass ein Einfluss des Materials der Mehrkammer-Biochips, insbesondere der (vorläufigen) Kulturkammern, auf die in einer solchen Zellkulturkammer erfolgenden Untersuchungen vorteilhaft reduziert werden kann. So haben die Erfinder unter anderem in Tests zur Wirkstoffadsorption mit Propiconazol und Troglitazon gefunden, dass PBT für Wirkstofftestungen von Substanzen bis zum logP von 3,72 (logP Propiconazol: 3,72; logP Troglitazon: 3,60) sehr gut geeignet ist. Nach einer Inkubationsdauer von 24h sind mindestens 80% der Ausgangskonzentration des Propiconazols bzw. des Troglitazons im Kulturmedium nachweisbar.

Der erfindungsgemäße Grundkörper kann in einer weiteren Ausführung derart gestaltet sein, dass Abschnitte der Kanäle in der dem Rahmen abgewandten Seitenfläche des Bodens und/oder in der dem Boden gegenüberliegenden Seitenfläche des Grundkörpers ausgeformt sind. Bevorzugt können die Kanalabschnitte in der Seitenfläche oder in den Seitenflächen vollständig oder teilweise als vorläufige Kanalabschnitte ausgebildet sein, indem umlaufende Kanalbegrenzungsstege ausgeformt sind. Diese Kanalbegrenzungsstege sind in den Grundkörper eingelassen, schließen mit diesem ab oder ragen aus dem Grundkörper heraus. Die Kanalbegrenzungsstege weisen nach innen gerichtete Seitenflächen auf, die einen Kanalraum abgrenzen und somit als Kanalwandung fungieren. Auf der Oberseite (Stirnseite) der Kanalbegrenzungsstege verlaufen Auflageflächen, die zur Auflage einer (Abschluss-)Membran oder eines Kanaldeckels vorgesehen sind.

Verbunden sind die Kanäle jeweils mit Anschlüssen (z.B. Standard Luer Format) zum Zuführen beziehungsweise Abführen von Medien. Vorteilhaft sind diese Anschlüsse dem Boden gegenüberliegend ausgeformt.

In dem Boden kann beispielsweise ein Fenster vorhanden sein, das der optischen Detektion von Vorgängen in wenigstens einer der Kulturkammern dient.

Um einen Mehrkammer-Biochip gemäß der Erfindung zu erhalten, wird ein erfindungsgemäßer Grundkörper bereitgestellt. Dieser dient als Basis des Mehrkammer-Biochips und erlaubt vorteilhaft sowohl eine effiziente Herstellung als auch eine flexible Anpassung an die jeweiligen Anforderungen.

Bei einem fertig montierten und gebrauchsfertigen Mehrkammer-Biochip ist auf die erste Auflagefläche des ersten Stegs eine erste Trennmembran aufgelegt und mit dieser verbunden. Dabei ist durch den ersten Steg und die erste Trennmembran eine untere Kulturkammer bereitgestellt. Je nach Ausführungsform des Grundkörpers ist gegebenenfalls auf einer zweiten Auflagefläche eines zweiten Stegs eine zweite Trennmembran aufgelegt und mit dem Steg verbunden. Zwischen der ersten Trennmembran und der zweiten Trennmembran ist in diesem Falle eine mittlere Kulturkammer bereitgestellt. Auf dem Rahmen ist eine Abschlussmembran aufgelegt und mit dem Rahmen verbunden. Diese Abschlussmembran begrenzt eine obere Kulturkammer, die je nach Ausführung des Grundkörpers zwischen der ersten Trennmembran und der Abschlussmembran oder zwischen der zweiten Trennmembran und der Abschlussmembran bereitgestellt ist.

Optional ist eine zusätzliche Abschlussmembran vorhanden, die auf der dem Rahmen abgewandten Seitenfläche des Bodens aufgelegt und mit diesem verbunden ist.

Als Trennmembranen dienen vorzugsweise Folien, die je nach Material, Dicke und Fertigung in vorgegebenen Graden flexibel integriert werden können und die durchlässig als auch undurchlässig für Gase, Flüssigkeiten, Partikel und/oder komplexere Moleküle (semipermeabel) sein können. Bevorzugte, aber nicht ausschließliche Materialien für die Trennmembranen sind Polyethylenterephthalat oder Polycarbonat. Bevorzugt weisen die Membranen Poren mit einer Größe zwischen 0.4 µm und 8 µm auf und haben eine Dicke zwischen 5 und 50 µm, bevorzugt 10 und 20 µm, besonders bevorzugt 12 µm. Die Trennmembranen können mindestens transluzent oder bevorzugt transparent für mindestens einen ausgewählten Wellenlängenbereich sein, um eine verbesserte optische Detektion von Vorgängen in wenigstens einer der Kulturkammern zu ermöglichen. Auch die Abschlussmembranen (zum Teil auch als Bondingfolien bezeichnet) sind vorzugsweise flexibel integrierbar und können entsprechend sperrend oder (halb-)durchlässig für bestimmte Stoffklassen gewählt sein. Die Abschlussmembranen können zudem transparent für mindestens einen ausgewählten Wellenlängenbereich sein, um eine optische Detektion von Vorgängen in wenigstens einer der Kulturkammern zu ermöglichen. In einigen Ausführungen können die Abschlussmembranen als transparente Abschluss-Folien, beispielsweise als Polycarbonatfolien oder Polyethylenterephthalat-Folien, ausgebildet sein. Auch Glas oder Polystyrene oder COC/COP sind mögliche Materialien, aus denen die Abschlussmembranen ausgeführt sein können. Die Abschlussmembranen (bzw. Abschluss-Folien) können zudem als Kanaldeckel fungieren, indem diese sich über optional vorhandene Kanalbegrenzungsstege erstrecken und auf deren Auflageflächen aufliegen und mit diesen gas- und flüssigkeitsdicht verbunden sind.

In einer besonderen Ausführung des Mehrkammer-Biochips kann wenigstens eine der Trennmembranen Vertiefungen aufweisen, die auch als (Mikro-)kavitäten bezeichnet werden. In diesen (Mikro-)kavitäten können Zellen, Zellverbünde, Sphäroide und/oder Organoide angesiedelt und/oder kultiviert werden.

Ein Anwendungsbeispiel ist hier die verbesserte/verlängerte Kultur von Sphäroiden und Organoiden über einen Zeitraum von bis zu 4 Tagen im Vergleich zur statischen Zellkultur.

Bei der Ausführung des Mehrkammer-Biochips mit Mikrokavitäten können Sphäroide und Organoide unter durchströmten (mikrofluidischen) Zellkulturbedingungen immobilisiert kultiviert werden, ohne dass eine zusätzliche Einbettung in (Hydro-)Gele, die meist aus Proteinen der extrazellulären Matrix in Einzelform oder Mischformen bestehen, notwendig wäre. Die Mikrokavitäten können zwischen 500 und 1500 µm im Durchmesser betragen, bevorzugterweise 800 µm. Die gel-freie Kultur erlaubt eine bessere optische Analyse während der Kulturzeit. Zudem erlaubt die gel-freie Kultur eine schonendere und zerstörungsfreie Rückgewinnung des intakten Zellgewebes, insbesondere von Sphäroiden und Organoiden aus dem Biochip für weiterführende Analysen wie beispielsweise Gewebeschnitte, Immunfluoreszenzfärbungen, Durchflusszytometrie, ELISA-basierte Assays oder eine Gewebelyse für DNA-/RNA-Analysen und Western Blot-Analysen.

Die gel-freie immobilisierte Kultur der Sphäroide und Organoide ermöglicht ferner eine erleichterte Co-Kultur mit Blutgefäßgewebe-Anordnungen (Vaskularisierung). Entweder auf unterschiedlichen Trennmembranen (indirekte Vaskularisierung) oder auf derselben (Mikro-)kavitäten-Trennmembran oder planen Trennmembran (direkte Vaskularisierung). Darüber hinaus ist es möglich, immobilisierte und vaskularisierte Sphäroide und Organoide mit Immunzellen im Kulturmedium direkt zu umspülen. Die gel-freie Kultur von Sphäroiden und Organoiden erleichtert hierbei erheblich das Einwandern von Immunzellen in das Gewebe der Sphäroide und Organoide.

Die Kultur unter durchströmten und /oder gelfreien Zellkulturbedingungen ermöglicht eine bessere Aufrechterhaltung der Vitalität biologischer Strukturen unter anderem infolge verbesserter Nährstoff-und Sauerstoffbedingungen, insbesondere von Sphäroiden, als unter vergleichbaren statischen Zellkulturbedingungen. Dadurch ist es möglich derartige biologische Strukturen länger für Testzwecke funktional zu erhalten.

Um einem Nutzer eine breite Auswahl an Verwendungsmöglichkeiten zu bieten, kann ein Set für einen erfindungsgemäßen Mehrkammer-Biochip bereitgestellt sein, das einen wie oben beschriebenen Grundkörper umfasst. Weiterhin ist in einem solchen Set mindestens eine erste Trennmembran vorhanden, die zum Auflegen auf den ersten Steg und zum Abschließen der unteren Kulturkammer dient. Optional ist dem Set mindestens eine zweite Trennmembran beigelegt, die zum Auflegen auf den zweiten Steg und zum Abschließen der mittleren Kulturkammer dient, falls der dem Set beigelegte Grundkörper einen zweiten Steg aufweist. Außerdem ist dem Set eine Abschlussmembran beigefügt, die zum Auflegen auf den Rahmen und zum Abschließen des verbliebenen Innenraums als eine obere Kulturkammer dient.

Ergänzend kann eine zusätzliche Abschlussmembran enthalten sein, die zum Auflegen auf die dem Rahmen abgewandte Seitenfläche des Bodens bestimmt ist.

Ein erfindungsgemäßes Set kann beispielsweise direkt einem Nutzer zur Verfügung gestellt oder einem Dienstleister geliefert werden, der im Auftrag und nach Maßgabe zum Beispiel des Nutzers die Montage der Komponenten des Sets vornimmt.

Vorteilhaft ermöglicht die Bereitstellung eines solchen Sets auch die Einbringung von biologischem Material, beispielsweise größerer Organoide oder Zellverbände oder Gewebestücke oder mehrzelliger Organismen, beispielsweise Parasiten, welche aufgrund ihrer Größe nicht durch die vorhandenen Kanäle in die Kammern eingespült werden können. Solches Material kann direkt in steriler Umgebung in eine noch offene vorläufige Kulturkammer appliziert werden, welche daran anschließend mit einer Trennmembran oder einer Abschlussmembran z.B. mittels eines adhäsiven Verfahrens verschlossen wird.

Die Herstellung des Mehrkammer-Biochips erfolgt, indem ein Grundkörper bereitgestellt und auf den ersten Steg die erste Trennmembran aufgelegt wird. Diese wird mit dem ersten Steg dicht verbunden. Gegebenenfalls kann vor Auflegen der ersten Trennmembran ein biologisches Material in die untere vorläufige Kulturkammer eingebracht werden wie weiter oben beschrieben.

Unter einem dichten Verbinden wird im Sinne der Beschreibung verstanden, dass eine flächige oder linienförmige Verbindung geschaffen ist, die insbesondere flüssigkeits- und gasdicht ist, sodass eine jeweils durch die Membran begrenzte Kulturkammer oder ein durch die Membran begrenzter Kanal den Durchfluss eines Mediums unter einem gewissen statischen oder dynamischen Arbeitsdruck sicher standhält.

Ein Verbinden erfolgt vorteilhaft aber nicht ausschließlich, indem ein gerichtet und gesteuert geführtes Bündel einer energiereichen Strahlung entlang der herzustellenden Verbindungsnaht oder Verbindungsfläche geführt wird. Die energiereiche Strahlung ist insbesondere eine Laserstrahlung, deren Wellenlänge und Intensität auf die zu verbindenden Materialien abgestimmt wird.

Ist die erste Trennmembran auf dem ersten Steg angebracht, wird gegebenenfalls die zweite Trennmembran auf den zweiten Steg aufgelegt und dicht mit diesem verbunden. Gegebenenfalls kann wiederum vor Auflegen der zweiten Trennmembran ein biologisches Material in die mittlere vorläufige Kulturkammer eingebracht werden. In entsprechender Weise erfolgt ein Auflegen der Abschlussmembran auf den Rahmen und ein dichtes Verbinden von Abschlussmembran und Rahmen. Optional wird die zusätzliche Abschlussmembran auf die dem Rahmen abgewandte Seitenfläche des Bodens aufgelegt und dicht mit diesem verbunden.

Da jede vorläufige Kulturkammer, und im Ergebnis jede der entstehenden Kulturkammern, von mindestens einem Kanal kontaktiert ist, kann in jede der Kulturkammern des Mehrkammer-Biochips ein Medium zugeführt und/oder abgeleitet werden.

Die vorstehend beschriebene Vielzahl von konkreten Ausführungsmöglichkeiten des erfindungsgemäßen Mehrkammer-Biochips erlaubt vorteilhaft eine erhebliche Anzahl von Möglichkeiten zur Verwendung eines solchen Mehrkammer-Biochips. Den bestimmungsgemäßen Verwendungen ist gemein, dass diese mindestens die folgenden Schritte umfassen. Zunächst wird ein erfindungsgemäßer Mehrkammer-Biochip ausgewählt und bereitgestellt. Die Auswahl kann beispielsweise erfolgen hinsichtlich der vorhandenen Anzahl an Kulturkammern und/oder hinsichtlich der Wahl und/oder der Kombination der Trennmembran(en). Um während seiner Verwendung unerwünschte Wechselwirkungen und Kontaminationen zu vermeiden, kann der Mehrkammer-Biochip anschließend sterilisiert werden. Einer Sterilisierung gleichgestellt ist auch eine Montage steriler Komponenten unter erhöhten Reinheitsbedingungen. Anschließend können in die vorhandenen Kulturkammern zum Beispiel Medien, Zellen, Mikroorganismen, Sphäroide und/oder Organoide und/oder Zellverbände und/oder Gewebestücke oder mehrzellige Organismen eingebracht werden.

In einer spezifischen Ausgestaltung einer Verwendung des Mehrkammer-Biochips beziehungsweise in einer seiner bereitgestellten Konfigurationen kann in wenigstens einer der Kulturkammern ein Hydrogel, vorzugsweise bestehend aus Komponenten der extrazellulären Matrix, wie z.B. Kollagenen, Fibronektin, Lamininen etc. eingebracht werden, beziehungsweise eingebracht sein.

Der erfindungsgemäße Mehrkammer-Biochip kann beispielsweise verwendet werden, um in wenigstens einer seiner Kulturkammern Sphäroide und/oder Organoide zu erzeugen und/oder zu kultivieren, indem diese in den (Mikro-)kavitäten einer der Membranen angesiedelt werden.

Der erfindungsgemäße Mehrkammer-Biochip kann ferner verwendet werden für Testungen von Zellen, Zellkulturen, Organoiden oder Sphäroiden mit verschiedenen Substanzen, Wirkstoffen, Nanomaterialien, Mikroorganismen, Vektoren, Antikörpern etc.

Die Erfindung ist aufgrund ihres Aufbaus auf Basis des erfindungsgemäßen Grundkörpers leicht für einen Nutzer zu verwenden. Die Montage eines Mehrkammer-Biochips ist gegenüber dem Stand der Technik erheblich vereinfacht und signifikant weniger fehleranfällig. Außerdem ist aufgrund der einstückigen Ausführung des Grundkörpers eine effiziente Herstellung sowie eine vielfältige Kombinierbarkeit mit verschiedensten Trennmembranen und/oder Abschlussmembranen möglich. Die Verwendung beispielsweise des Verfahrens des Laserbondens zum dichten Verbinden der Membranen erlaubt vorteilhaft, auf Klebstoffe oder andere Adhäsive zu verzichten.

Ein erfindungsgemäßer Mehrkammer-Biochip ist je nach spezifischer Ausführung beispielsweise für Wirkstofftestungen oder die Etablierung und Charakterisierung von Organ- oder Organoid-Modellen und Krankheits- und Infektionsmodellen verwendbar. Für Wirkstofftestungen ist beispielsweise denkbar, eine Immunantwort der kultivierten Zellen auf eine Substanzgabe hin zu untersuchen. Dabei bestehen unterschiedliche Möglichkeiten: die Substanz kann beispielsweise in die Kammer gegeben werden, in der die zu testenden Zellen wachsen. Der Einfluss der Substanz auf die Zellen, kann dann beispielsweise durch mikroskopische Beobachtung der Zellen oder durch Untersuchung des Zellkulturmediums z.B. auf von den Zellen in das Medium abgegebene Botenstoffe, Marker etc. ausgelesen werden. Die Substanz kann alternativ auch in die den zu testenden Zellen gegenüberliegende Kammer gegeben werden, um beispielsweise einen Einfluss von in dieser den zu testenden Zellen gegenüberliegenden Kammer wachsenden Zellen auf die Wirkung der Substanz bei den zu testenden Zellen der anderen Kammer zu untersuchen, die beispielsweise eine Substanzwirkung abschwächen oder potenzieren könnten (Gradientenbildung). Es können weiterhin Sphäroide und/oder Organoide mit und ohne Immunzellpopulationen eingespült/perfundiert werden. Die Immunzellen können in die Kammer mit den Sphäroiden oder Organoiden eingespült/perfundiert werden oder aber bevorzugterweise über eine Blutgefäßstruktur in einer der angrenzenden Kammern eingespült/perfundiert werden. Zudem können die Immunzellen gewebeständig in Blutgefäßstruktur und/oder die Sphäroide/Organoide integriert werden. Sphäroide beziehungsweise Organoide können zudem durch das Einbringen von Blutgefäßzellen wie Endothelzellen allein oder Endothelzellen in Kombination mit, aber nicht ausschließlich Perizyten und glatten Muskelzellen vaskularisiert werden. Um beispielsweise ein von Blutgefäßen durchzogenes Gewebe nachzuempfinden, können die oberen und unteren Wände der mittleren Kulturkammer mit Endothelzellen allein oder in Kombination mit Perizyten und glatten Muskelzellen ausgekleidet werden, die durch den zuführenden Kanal eingebracht werden, während in die untere und in die obere Kulturkammer organspezifische Epithelzellen eingebracht werden. In einer weiteren Ausführung können die Endothelzellen allein oder in Kombination mit Perizyten und glatten Muskelzellen jedoch auch an die oberen und unteren Wände der oberen oder unteren Kammer durch den jeweils zuführenden Kanal eingebracht werden und in der mittleren Kammer Epithelgewebe in Form geschichteter Zelllayer oder in Form von Sphäroiden und Organoiden integriert werden.

Die Erfindung wird nachfolgend anhand von Ausführungsbeispielen und Abbildungen näher erläutert. Dabei zeigen:
- Fig. 1: eine schematische Darstellung eines Ausführungsbeispiels eines erfindungsgemäßen Grundkörpers in einer perspektivischen Ansicht;
- Fig. 2: eine schematische Darstellung eines Ausführungsbeispiels von Kanälen im Boden eines erfindungsgemäßen Grundkörpers;
- Fig. 3: eine schematische Darstellung eines Ausführungsbeispiels eines erfindungsgemäßen Sets für die Bereitstellung eines Mehrkammer-Biochips (Explosionsansicht);
- Fig. 4: einen seitlichen Schnitt durch einen erfindungsgemäßen Mehrkammer-Biochip mit drei Kulturkammern sowie eine schematische Darstellung einer Vorrichtung zum Betrieb des Mehrkammer-Biochips; und
- Fig.5: eine mögliche Verwendung eines erfindungsgemäßen Mehrkammer-Biochips.

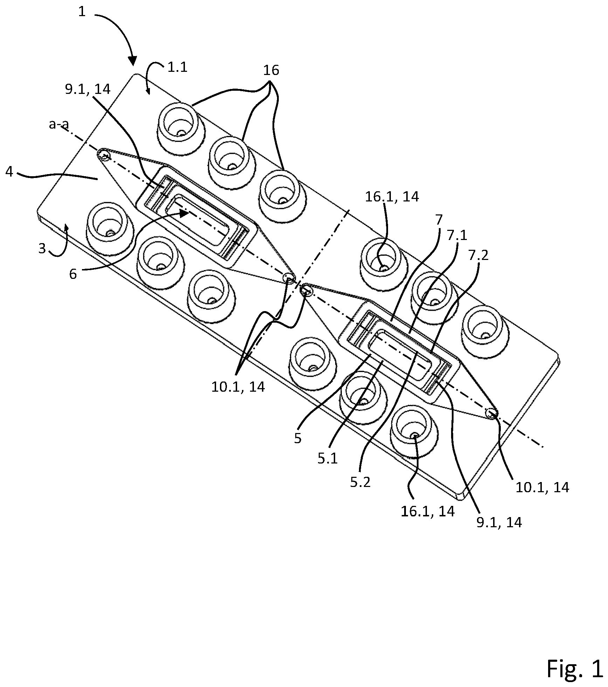

Im Folgenden wird die Erfindung anhand von Ausführungsbeispielen beschrieben, bei denen auf einem Boden 3 eines Grundkörpers 1 jeweils zwei Innenräume 6 durch eine rechteckige flächige Ausprägung eines Rahmens 4 abgegrenzt sind. Wie im Folgenden noch ausführlich erläutert wird, sind die Innenräume 6 gestuft untergliedert, derart, dass sie jeweils eine (vorläufige) Mehrkammer-Kavität 2.1 ausbilden. Durch den Grundkörper 1 der Figuren 1, 2 und 3 kann somit ein Mehrkammer-Biochip 2 bereitgestellt werden, beziehungsweise ist ein Mehrkammer-Biochip 2 bereitgestellt, der zwei Mehrkammer-Kavitäten 2.1 aufweist (wobei jedoch beide Mehrkammer-Kavitäten 2.1 funktional jeweils einen vollständigen Mehrkammer-Biochip bilden). Um die Übersichtlichkeit der Abbildungen zu verbessern, werden in den Figuren 1, 2 und 3 beide vorhandenen Mehrkammer-Kavitäten 2.1 genutzt, um die Elemente einer einzelnen Mehrkammer-Kavität 2.1 bzw. eines Mehrkammer-Biochips 2 mit nur einer Mehrkammer-Kavität 2.1 zu bezeichnen. Die Beschreibung bezieht sich dabei nur auf eine Mehrkammer-Kavität 2.1.

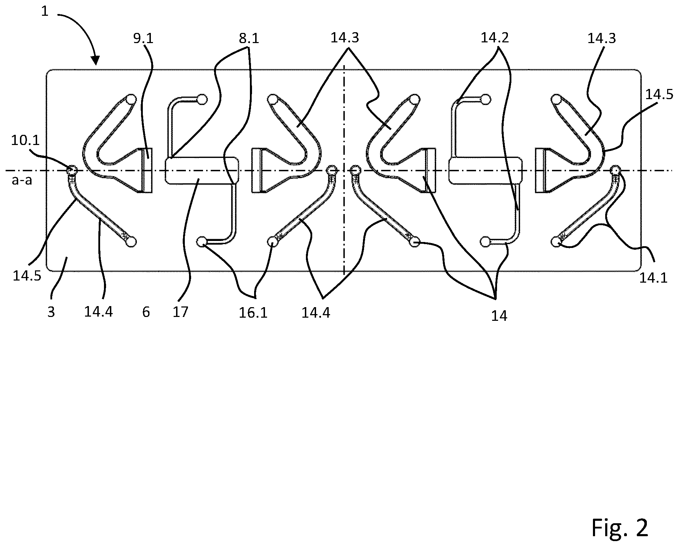

Ein erfindungsgemäßer Grundkörper 1 ist einstückig aus einem biokompatiblen Material, insbesondere aus einem biokompatiblen spritzgussfähigen Kunststoff, gebildet (Fig. 1). Ausgehend von einem Boden 3 (siehe auch Fig. 2) ist ein auf seiner dem Boden abgewandten Seite offener Rahmen 4 ausgebildet. Der Rahmen 4 ist flächig ausgeprägt und umgrenzt dabei einen Innenraum 6.

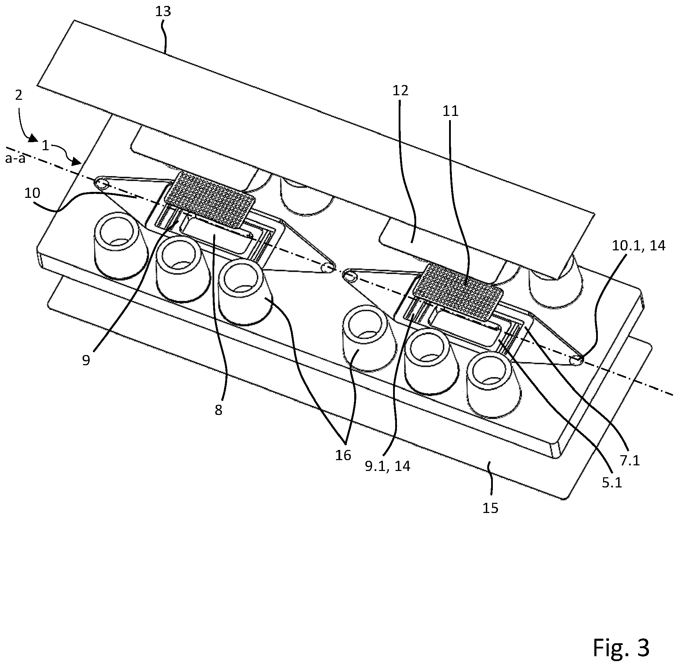

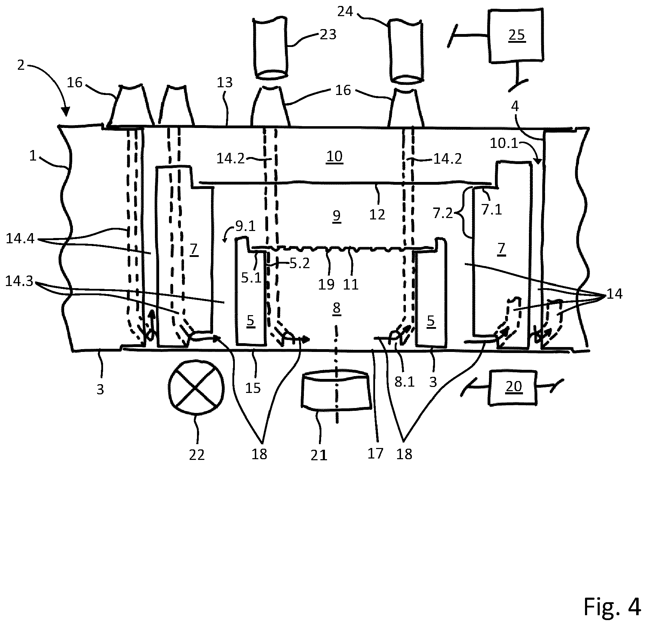

Innerhalb des Innenraums 6 ist ein um eine erste Fläche umlaufender erster Steg 5 in Form einer in dem Material des Rahmens 4 vorhandenen Stufe ausgebildet. Die Höhe des ersten Stegs 5 ist geringer als die Höhe des Rahmens 4. Der erste Steg 5 weist eine dem Innenraum 6 zugewandte erste Seitenfläche 5.2 und dem Boden 3 gegenüberliegend eine erste Auflagefläche 5.1 auf. Die erste Auflagefläche 5.1 ist zur Auflage einer ersten Trennmembran 11 (siehe Fig. 3, 4) ausgebildet. Durch die erste Seitenfläche 5.2 und die von dem ersten Steg 5 umfangene Fläche ist eine untere vorläufige Kulturkammer 8 umgrenzt. In dem Ausführungsbeispiel der Figuren 1 bis 3 ist die von dem ersten Steg 5 umfangene Fläche rechteckig.

Außerdem ist ein innerhalb des Innenraums 6 auf dem Boden 3 aufsetzender und um eine zweite Fläche umlaufender zweiter Steg 7 vorhanden, der ebenfalls als eine Stufe mit Material des Grundkörpers 1 ausgebildet ist (Fig. 1). Die von dem zweiten Steg 7 umfangene zweite Fläche ist größer als die von dem ersten Steg 5 umfangene erste Fläche und ebenfalls rechteckig. Die Höhe des zweiten Stegs 7 ist geringer als die Höhe des Rahmens 4, jedoch größer als die Höhe des ersten Stegs 5. Auf dem zweiten Steg 7 ist eine zweite Auflagefläche 7.1 ausgebildet. Die zweite Auflagefläche 7.1 ist zur Auflage einer zweiten Trennmembran 12 (siehe Fig. 3) ausgebildet. In dem Abschnitt zwischen der ersten Auflagefläche 5.1 des ersten Stegs 5 und der zweiten Auflagefläche 7.1 des zweiten Stegs 7 bildet der zweite Steg 7 eine dem Innenraum 6 zugewandte zweite Seitenfläche 7.2 aus. Durch die von dem zweiten Steg 7 umfangene zweite Fläche und die zweite Seitenfläche 7.2 ist eine mittlere vorläufige Kulturkammer 9 umgrenzt. Der verbleibende Innenraum 6 zwischen der zweiten Auflagefläche 7.1 und einer Oberseite 1.1 des Grundkörpers 1 bildet eine obere vorläufige Kulturkammer 10. Die Oberseite 1.1. ist dabei durch die dem Boden 3 gegenüberliegende Seitenfläche des Grundkörpers 1 gebildet.

Um die aus den vorläufigen Kulturkammern 8, 9, 10 im montierten Zustand des Mehrkammer-Biochips 2 resultierenden Kulturkammern 8, 9 und 10 mit Medien 18 (siehe Fig. 4) versorgen zu können, sind je zwei Kanäle 14 zwischen den Kulturkammern 8, 9 und 10 und der Oberseite 1.1 des Grundkörpers 1 ausgebildet. In der Figur 1 sind die Kanäle 14 als runde Durchbrüche 10.1, 16.1 sowie als Durchbrüche mit rechteckigen Querschnitten 9.1 zu sehen. Die Kanäle 14 führen von den Kulturkammern 8, 9 und 10 in Richtung Boden 3, von wo aus eine Verteilung hin zu Anschlüssen 16 erfolgt (vgl. Fig. 1, 3, 4).

Die Anschlüsse 16 setzen im gezeigten Ausführungsbeispiel auf der Oberseite 1.1 des Grundkörpers 1 auf. Im Ausführungsbeispiel ist je einer der Anschlüsse 16 für die Zuführung und für den Abtransport von Medien 18 durch jede der Kulturkammern 8, 9, 10 eingerichtet. Jede der Kulturkammern 8, 9, 10 eines betriebsbereiten Mehrkammer-Biochips 2 kann daher unabhängig von den anderen Kulturkammern 8, 9, 10 von einem Medium 18 durchströmt werden. Insbesondere kann jedes Medium 18 individuell für die jeweilige Kulturkammer 8, 9, 10 gewählt und mit einem individuell regelbaren Volumenstrom an die jeweilige Kulturkammer 8, 9, 10 angelegt werden.

Um während des Betriebs des Mehrkammer-Biochips 2 eine optische Detektion von Vorgängen mindestens in der unteren Kulturkammer 8 zu ermöglichen, ist in dem Boden 3 ein Fenster 17 ausgebildet (Fig.4). Die Fläche des Fensters 17 ist deckungsgleich mit der von dem ersten Steg 5 umlaufenen Fläche.

Der beispielhafte Verlauf der Kanäle 14 sowie deren Verbindung mit den jeweiligen Anschlüssen 16 ist in der **Fig. 2** mit einer Darstellung der nach außen weisenden Seitenfläche des Bodens 3 dargestellt. Zum Antransport und Abtransport eines Mediums 18 durch die untere Kulturkammer 8 sind zwei untere Kanäle 14.2 vorhanden, die durch zwei einander schräg gegenüber angeordnete Versorgungsmündungen 8.1 in die untere Kulturkammer 8 münden. Zur Versorgung der mittleren Kulturkammer 9 mit einem weiteren Medium 18 sind zwei mittlere Kanäle 14.3 vorgesehen, die abschnittsweise als einander gegenüberliegende, zwischen dem ersten Steg 5 und dem zweiten Steg 7 angeordnete, rechteckige Versorgungsdurchbrüche 9.1 ausgeformt sind (siehe auch Fig. 1). Die obere Kulturkammer 10 wird durch zwei obere Kanäle 14.4 mit noch einem weiteren Medium 18 versorgt., indem diese die obere Kulturkammer 10 über die jeweils am weitesten außen liegenden runden Versorgungsdurchbrüche 10.1 kontaktieren.

Die jeweils mit einem runden Querschnitt gezeigten Anschlussdurchbrüche 16.1 stellen sodann die jeweiligen Verbindungen zu den Anschlüssen 16 her.

Im Ausführungsbeispiel der Fig. 2 sind Abschnitte 14.1 der Kanäle 14 zwischen den Versorgungsmündungen 8.1 sowie der Versorgungsdurchbrüche 9.1, 10.1 einerseits und den jeweils zugehörigen Anschlussdurchbrüchen 16.1 andererseits vorhanden, die als vorläufige Kanalabschnitte 14.1 gearbeitet sind. Die vorläufigen Kanalabschnitte 14.1 werden durch in den Grundkörper 1 eingearbeitete Vertiefungen gebildet. Im Ausführungsbeispiel sind die Vertiefungen nutförmig angelegt mit einem rechteckigen Querschnitt. Selbstverständlich sind auch andere Arten von Vertiefungen möglich mit anderen Querschnitten wie beispielsweise einem halbkreisförmigen Querschnitt. Die Vertiefungen weisen innere Kanalwandungen 14.5 auf, die jeweils einen Anschlussdurchbruch 16.1 und einen Versorgungsdurchbruch 9.1, 10.1, beziehungsweise, im Falle der unteren Kulturkammer 8, die beiden zugehörigen Anschlussdurchbrüche 16.1 und das Fenster 17 mit den beiden schräg gegenüberliegenden Versorgungsmündungen 8.1, umschließen. Die Kanalwandungen 14.5 schließen bündig ab mit der von dem Rahmen 4 wegweisenden Seitenfläche des Bodens 3. Durch Aufbringung eines Kanaldeckels können die vorläufigen Kanalabschnitte 14.1 verschlossen und somit der funktionale Zustand dieser Kanalabschnitte 14.1 hergestellt werden. Im Ausführungsbeispiel der Fig. 3 und 4 wird eine untere Abschlussmembran 15 auf die von dem Rahmen 4 wegweisenden Seitenfläche des Bodens 3 aufgebracht und nahtförmig entlang der Kanalwandungen 14.5 flüssigkeitsdicht mit dem Boden 3 verbunden. Die Verbindung erfolgt vorzugsweise mittels Laserschweißens entlang der herzustellenden Verbindungsnaht, kann aber beispielsweise auch durch Kleben oder Quellschweißen erfolgen. Durch die flüssigkeitsdichte Anbindung der Abschlussmembran 15 erhalten die vorläufigen Kanalabschnitte 14.1 ihren funktionalen Zustand.

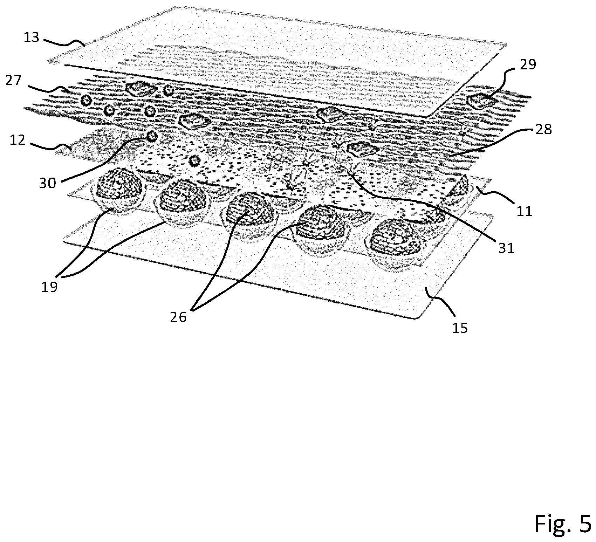

In der **Fig. 3** ist beispielhaft ein Set für einen Mehrkammer-Biochip 2 gezeigt, wobei die Abbildung auch als eine Explosionsdarstellung der Komponenten eines Ausführungsbeispiels eines Mehrkammer-Biochips 2 angesehen werden kann. Als zentrales Element ist ein erfindungsgemäßer Grundkörper 1 vorhanden. Eine erste flexible Trennmembran 11 ist zum Auflegen auf die erste Auflagefläche 5.1 des ersten Stegs 5 vorgesehen. Wird die erste Trennmembran 11 dort befestigt, schließt diese eine untere Kulturkammer 8 ab. Die erste Trennmembran 11 ist beispielsweise mit Mikrokavitäten 19 versehen, in die beispielsweise Sphäroide oder Organoide eingebracht und/oder dort kultiviert werden können (siehe Fig. 5). Eine zweite flexible Trennmembran 12 dient zum Auflegen auf die zweite Auflagefläche 7.1 des zweiten Stegs 7 und schließt eine mittlere Kulturkammer 9 ab. Auf den Rahmen 4 kann eine ebenfalls bereitgestellte transparente Abschlussmembran 13 aufgelegt werden, die im montierten Zustand zum Abschließen des verbliebenen Innenraums 6 als eine obere Kulturkammer 10 zwischen der zweiten Trennmembran 12 und der Abschlussmembran 13 dient.

Um auch die im Boden 3 ausgebildeten Kanalabschnitte 14.1 sowie das Fenster 17 dicht abzuschließen, ist eine zusätzliche transparente Abschlussmembran 15 vorhanden, die, wie schon weiter oben erläutert, auf dem Boden 3 aufgebracht wird und die jeweiligen Kanalabschnitte 14.1 sowie das Fenster 17 überspannt und abdichtet.

Ein erfindungsgemäßer Mehrkammer-Biochip 2 mit einer unteren Kulturkammer 8, einer mittleren Kulturkammer 9 und einer oberen Kulturkammer 10 ist in **Fig. 4** in einer seitlichen Schnittdarstellung gezeigt. Die Zeichenebene der Fig. 4 entspricht der Linie a-a der Figur 3, wobei in der Figur 4 aus Übersichtlichkeitsgründen nur die linke Hälfte des Mehrkammer-Biochips der Fig. 3 gezeigt wird.

In Figur 4 gut zu erkennen ist der monolithische Aufbau des Grundkörpers 1 des Mehrkammer-Biochips 2. Ausgehend von einem Boden 3 ist ein flächig ausgeprägter Rahmen 4 ausgebildet, der auf seiner dem Boden 3 abgewandten Seite offen ist. Innerhalb des Rahmens 4 ist ein um eine erste Fläche umlaufender erster Steg 5 in Form einer in dem Material des Rahmens 4 vorhandenen Stufe ausgebildet. Die Höhe des ersten Stegs 5 ist geringer als die Höhe des Rahmens 4. Der erste Steg 5 weist eine erste Seitenfläche 5.2 und dem Boden 3 gegenüberliegend eine erste Auflagefläche 5.1 auf. Im Bereich der von dem ersten Steg 5 umfangenen Fläche weist der Boden ein Fenster 17 auf. Weiter ist ein auf dem Boden 3 aufsetzender und um eine zweite Fläche umlaufender zweiter Steg 7 vorhanden, der ebenfalls als eine Stufe mit Material des Grundkörpers 1 ausgebildet ist. Die Höhe des zweiten Stegs 7 ist geringer als die Höhe des Rahmens 4, jedoch größer als die Höhe des ersten Stegs 5. Auf dem zweiten Steg 7 ist eine zweite Auflagefläche 7.1 ausgebildet. Zwischen der ersten Auflagefläche 5.1 des ersten Stegs 5 und der zweiten Auflagefläche 7.1 des zweiten Stegs 7 bildet der zweite Steg 7 eine dem Innenraum 6 zugewandte zweite Seitenfläche 7.2 aus.

Auf der ersten Auflagefläche 5.1 ist eine erste Trennmembran 11 und auf der zweiten Auflagefläche 7.1 ist eine zweite Trennmembran 12 flüssigkeitsdicht aufgebracht. Die erste Trennmembran 11 weist Mikrokavitäten 19 auf. Auf der dem Rahmen 4 abgewandten Seitenfläche des Bodens 3 ist eine untere Abschlussmembran 15 und auf der Oberseite des Rahmens ist eine obere Abschlussmembran 13 flüssigkeitsdicht aufgebracht. Durch die erste Seitenfläche 5.2, die erste Trennmembran 11 und untere Abschlussmembran 15 ist eine untere Kulturkammer 8 abgegrenzt. Durch die zweite Seitenfläche 7.2, die erste Trennmembran 11 und die zweite Trennmembran 12 ist eine mittlere Kulturkammer 9, und durch den verbleibenden Rahmen 4, die zweite Trennmembran 12 und die obere Abschlussmembran 13 ist eine obere Kulturkammer 10 abgegrenzt.

Gut zu erkennen sind die Funktionen der Membranen 11, 12, 13 und 15 sowohl zur Abgrenzung der Kulturkammern 8, 9 und 10 gegeneinander beziehungsweise gegen die Umgebung, als auch zur Bereitstellung gewünschter Optionen hinsichtlich des Austauschs von Molekülen und/oder Zellen zwischen den Kulturkammern 8, 9 und 10.

In den Beispielen der Figuren 3 und 4 weist der Mehrkammer-Biochip 2 drei Kammern auf. Die untere Kammer 8 hat eine nutzbare Grundfläche von ca. 42 mm², eine Höhe von 0,5 mm und ein Volumen von 60 mm³ inklusive des Volumens der zuführenden und abführenden Kanäle 14. Im Bereich der Versorgungsmündungen 8.1 weisen die zuführenden und abführenden Kanäle 14 einen Durchmesser von etwa 0,5 mm auf. Die mittlere Kammer 9 hat eine nutzbare Grundfläche von 160 mm², eine Höhe von 1,1 mm und ein Volumen von knapp 200 mm³ inklusive des Volumens der zuführenden und abführenden Kanäle 14. Die zuführenden und abführenden Kanäle 14 sind im Bereich der Versorgungsdurchbrüche 9.1 rechteckig und 2 mm breit. Die obere Kammer 10 hat eine nutzbare Grundfläche von 216 mm², eine Höhe von 0,7 mm und ein Volumen von rund 150 mm³ inklusive des Volumens der zuführenden und abführenden Kanäle 14. Die zuführenden und abführenden Kanäle 14 weisen im Bereich der Versorgungsdurchbrüche 10.1 einen Durchmesser von 0,8 mm auf. Alle genannten eindimensionalen Angaben können variieren und beispielsweise eine Variabilität von ± 0,5 mm aufweisen, was in Änderungen der Größe der Flächen und Volumina resultieren kann.

Verschiedene Medien 18 (durch Pfeile versinnbildlicht) können entlang der zugeordneten Kanäle 14 in die jeweiligen Kulturkammern 8, 9, 10 hinein und wieder herausfließen, wobei die Versorgung mit Medien 18 in den Kulturkammern 8, 9, 10 unabhängig voneinander erfolgen kann (Fig. 4). Das Anschlussprinzip ist in Figur 4 am Beispiel der unteren Kulturkammer 8 gezeigt: die zugehörigen Anschlüsse 16, über denen jeweils eine Zuleitung 23 und eine Ableitung 24 angeordnet ist, sind mit unteren Kanälen 14.2 verbunden, welche über Versorgungsmündungen 8.1 in die untere Kulturkammer 8 münden und derart die untere Kammer 8 mit einem Medium 18 versorgen. Die unteren Kanäle 14.2 verlaufen zum Teil versetzt zu der Zeichenebene und sind daher teilweise gestrichelt eingezeichnet.

In analoger Weise können die mittlere Kulturkammer 9 über die mittleren Kanäle 14.3 und die obere Kulturkammer 10 über die oberen Kanäle 14.4 mit Medium 18 versorgt werden. Der Mehrkammer-Biochip 2 kann beispielsweise mittels einer Vorrichtung wie einem Lesegerät oder einem Mikroskop betrieben werden, die ein Objektiv 21 aufweist, das auf das Fenster 17 gerichtet ist und mittels dem Vorgänge in dem Mehrkammer-Biochip 2 beobachtet und optional erfasst, gespeichert und ausgewertet werden können. Dazu kann weiterhin eine Lichtquelle 22 vorhanden sein, um den Mehrkammer-Biochip 2 in gewünschter Weise zu beleuchten. Außerdem kann eine Pumpe 25 angeordnet sein, die mit den Zuleitungen 23 und Ableitungen 24 in Verbindung steht, die ihrerseits an den entsprechenden Anschlüssen 16 angebracht sind. Die Pumpe 25 sowie optional die Lichtquelle 22 sind mittels einer Steuerung 20 ansteuerbar, sodass beispielsweise eine Perfusion der Kulturkammern 8, 9, 10 gesteuert vorgenommen werden und dabei optisch überwacht werden kann. Die Steuerung 20, die beispielsweise durch einen Rechner realisiert ist, kann neben der Erzeugung von Steuerbefehlen optional auch optisch erfasste Daten speichern und/oder auswerten. Möglich ist zum Beispiel eine Regelung der Pumpleistungen für die einzelnen Kulturkammern 8, 9, 10 in Abhängigkeit von den optisch erfassten Daten mittels der Steuerung 20.

Die Erfindung ermöglicht vorteilhaft den Aufbau komplexer biologischer Modelle. Beispielsweise können mikrofluidische Kulturen von Sphäroiden 26 und/oder Organoiden 26 mit integriertem Blutgefäß und Immunzellzirkulation realisiert werden.

So kann ein Modell zur Untersuchung von Bauchspeicheldrüsenkrebs (PDAC, pancreatic ductal adenocarcinoma) erstellt sein (Fig. 5). Dazu wird die untere Kulturkammer 8 mit einer lichten Höhe von 0,5 mm zwischen unterer Abschlussmembran 15 und erster Trennmembran 11 bereitgestellt. Die erste Trennmembran 11 ist porös und auf ihrer zur mittleren Kulturkammer 9 weisenden Seitenfläche mit Mikrokavitäten 19 versehen, in denen Sphäroide 26 angesiedelt und kultiviert werden können. Die mittlere Kulturkammer 9 weist eine lichte Höhe von 1,1 mm auf, sodass Sphäroide 26 bis zu einer Größe von etwa 1 mm zerstörungsfrei eingebracht werden können. Entsprechend groß dimensioniert sind auch die Kanäle 14.

Die zweite Trennmembran 12 ist im Ausführungsbeispiel der Fig. 5 als eine poröse PET-Folie ausgeführt und deckt die mittlere Kulturkammer 9 ab. In der oberen Kulturkammer 10 ist eine Zellschicht 27, bestehend aus mikrovaskulären Pankreasendothelzellen 28 mit Makrophagen 29 ausgebildet. Der besseren Übersichtlichkeit halber ist die Zellschicht 27 beabstandet zur zweiten Trennmembran 12 dargestellt, in der Realität wächst die Zellschicht 27 adhärent auf der zweiten Trennmembran 12. Durch die zweite Trennmembran 12 hindurch können beispielsweise perfundierte Monozyten 30 und T-Zellen 31 in die mittlere Kulturkammer 9 gelangen.

Die obere Kulturkammer 10 besitzt eine lichte Höhe von 0,7 mm zwischen zweiter Trennmembran 7 und Abschlussmembran 13.

### Bezugszeichen

- 1: Grundkörper
- 1.1: Oberseite
- 2: Mehrkammer-Biochip
- 2.1: Mehrkammer-Kavität
- 3: Boden
- 4: Rahmen
- 5: erster Steg
- 5.1: erste Auflagefläche (erste Stirnseite)
- 5.2: erste Seitenfläche
- 6: Innenraum
- 7: zweiter Steg
- 7.1: zweite Auflagefläche (zweite Stirnseite)
- 7.2: zweite Seitenfläche
- 8: untere (vorläufige) Kulturkammer
- 8.1: Versorgungsmündung
- 9: mittlere (vorläufige) Kulturkammer
- 9.1: Versorgungsdurchbruch
- 10: obere (vorläufige) Kulturkammer
- 10.1: Versorgungsdurchbruch
- 11: erste Trennmembran
- 12: zweite Trennmembran
- 13: obere Abschlussmembran (bzw. obere Bondingfolie)
- 14: Kanal
- 14.1: vorläufiger Kanalabschnitt
- 14.2: unterer Kanal
- 14.3: mittlerer Kanal
- 14.4: oberer Kanal
- 14.5: Kanalwandung
- 15: zusätzliche (untere) Abschlussmembran (bzw. untere Bondingfolie)
- 16: Anschluss
- 16.1: Anschlussdurchbruch
- 17: Fenster
- 18: Medium
- 19: Mikrokavität
- 20: Steuerung
- 21: Objektiv
- 22: Lichtquelle
- 23: Zuleitung
- 24: Ableitung
- 25: Pumpe
- 26: Sphäroid/Organoid
- 27: Zellschicht
- 28: (Pankreas-)Endothelzellen
- 29: Makrophagen
- 30: Monozyten
- 31: T-Zellen

## Patentansprüche

1. Grundkörper (1) eines Mehrkammer-Biochips (2), gebildet aus einem einzigen Werkstück (monolithisch) und aufweisend
- einen Boden (3) mit einem darauf befindlichen und auf seiner dem Boden (3) abgewandten Seite offenen Rahmen (4), wobei durch den Rahmen (4) mindestens ein Innenraum (6) umgrenzt ist;
- mindestens einen innerhalb des Innenraums (6) auf dem Boden (3) aufsetzenden und um eine erste Fläche umlaufenden ersten Steg (5) mit einer ersten Auflagefläche (5.1) und einer ersten Seitenfläche (5.2), deren Höhe geringer ist als die Höhe des Rahmens (4), wobei
durch die erste Seitenfläche (5.2) und die von dem ersten Steg (5) umfangene Fläche eine untere vorläufige Kulturkammer (8) umgrenzt ist und
der verbliebene Innenraum zwischen der ersten Auflagefläche (5.1) und der Oberseite des Rahmens (4) eine obere vorläufige Kulturkammer bildet;
- mindestens einen an einer Außenseite des Grundkörpers mündenden Kanal (14), der die untere vorläufige Kulturkammer (8) mit der Umgebung verbindet;
- mindestens einen weiteren an einer Außenseite des Grundkörpers mündenden Kanal (14), der die obere vorläufige Kulturkammer (10) mit der Umgebung verbindet.

2. Grundkörper (1) nach Anspruch 1, **gekennzeichnet durch**
- einen innerhalb des Innenraums (6) um eine zweite Fläche umlaufenden zweiten Steg (7), dessen Höhe geringer als die Höhe des Rahmens (4) und größer als die Höhe des ersten Stegs (5) ist,
wobei durch eine zweite Seitenfläche (7.2) des zweiten Stegs (7) und die von dem zweiten Steg (7) umfangenen Fläche eine mittlere vorläufige Kulturkammer (9) umgrenzt ist, und
- mindestens einen weiteren an einer Außenseite des Grundkörpers mündenden Kanal (14), der die mittlere vorläufige Kulturkammer (9) mit der Umgebung verbindet.

3. Grundkörper (1) nach einem der Ansprüche 1 oder 2, **dadurch gekennzeichnet, dass** das Material, aus dem der Grundkörpers (1) gefertigt ist, einen spritzgussfähigen, biokompatiblen Kunststoff, insbesondere Polybutylenterephthalat (PBT), umfasst.

4. Grundkörper (1) nach einem der vorhergehenden Ansprüche, **dadurch gekennzeichnet, dass** Abschnitte der Kanäle (14) in der dem Rahmen (4) abgewandten Seitenfläche des Bodens (3) ausgeformt und jeweils mit Anschlüssen (16) der Kanäle (14) zum Zuführen beziehungsweise Abführen von Medien verbunden sind.

5. Grundkörper (1) nach Anspruch 4, **dadurch gekennzeichnet, dass** die Abschnitte der Kanäle (14) vollständig oder teilweise als vorläufige Kanalabschnitte (14.1) ausgebildet sind.

6. Grundkörper (1) nach einem der vorhergehenden Ansprüche, **dadurch gekennzeichnet, dass** auf dem Boden (3) mindestens ein weiterer Rahmen (4) mit einem ersten Steg (5) und gegebenenfalls einem zweiten Steg (7) ausgebildet sind.

7. Mehrkammer-Biochip (2),
- mit einem Grundkörper (1) nach einem der Ansprüche 1 bis 6,
- mit einer auf den ersten Steg (5) aufgelegten und mit diesem verbundenen ersten Trennmembran (11), wobei
durch den ersten Steg (5) und die erste Trennmembran (11) eine untere Kulturkammer (8) bereitgestellt ist;
- gegebenenfalls mit einer auf den zweiten Steg (7) aufgelegten und mit diesem verbundenen zweiten Trennmembran (12), wobei
zwischen der ersten Trennmembran (11) und der zweiten Trennmembran (12) eine mittlere Kulturkammer (9) bereitgestellt ist; und
- mit einer auf einer Oberseite1.1 des Rahmens (4) aufgelegten und mit diesem verbundenen oberen Abschlussmembran (13), wobei
zwischen der oberen Abschlussmembran (13) und der ersten Trennmembran (11) beziehungsweise gegebenenfalls zwischen der der oberen Abschlussmembran (13) und der zweiten Trennmembran (12) und eine obere Kulturkammer (10) bereitgestellt ist; sowie
- optional einer auf der dem Rahmen (4) abgewandten Seitenfläche des Bodens (3) aufgelegten und mit diesem verbundenen unteren Abschlussmembran (15).

8. Mehrkammer-Biochip nach Anspruch 7, **dadurch gekennzeichnet, dass** wenigstens eine der Trennmembranen (11, 12) Mikrokavitäten (19) aufweist.

9. Verfahren zur Herstellung eines Mehrkammer-Biochips nach Anspruch 7 oder 8, umfassend die Schritte
- Bereitstellen eines Grundkörpers (1) nach einem der Ansprüche 1 bis 6,
- Auflegen der ersten Trennmembran (11) auf den ersten Steg (5) und dichtes Verbinden von erster Trennmembran (11) und erstem Steg (5);
- gegebenenfalls Auflegen der zweiten Trennmembran (12) auf den zweiten Steg (7) und dichtes Verbinden von zweiter Trennmembran (12) und zweitem Steg (7);
- Auflegen der oberen Abschlussmembran (13) auf den Rahmen (4) und dichtes Verbinden von oberer Abschlussmembran (13) und Rahmen (4);
- optional Auflegen der unteren Abschlussmembran (15) auf die dem Rahmen abgewandte Seitenfläche des Bodens (3) und dichtes Verbinden von Boden (3) und unterer Abschlussmembran (15).

10. Verfahren nach Anspruch 9, **dadurch gekennzeichnet, dass** das dichte Verbinden mittels eines gerichteten und gesteuert geführten Bündels einer energiereichen Strahlung erfolgt.

11. Set für einen Mehrkammer-Biochip (2) nach Anspruch 7, enthaltend
- einen Grundkörper (1) nach einem der Ansprüche 1 bis 7;
- mindestens eine erste Trennmembran (11), die zum Auflegen auf den ersten Steg (5) und zum Abschließen der unteren Kulturkammer (8) dient;
- optional mindestens eine zweite Trennmembran (12), die zum Auflegen auf den zweiten Steg (7) und zum Abschließen der mittleren Kulturkammer (9) dient; und
- eine obere Abschlussmembran (13), die zum Auflegen auf den Rahmen (4) und zum Abschließen der oberen Kulturkammer (10) dient.

12. Set nach Anspruch 11, **gekennzeichnet durch** eine zusätzliche untere Abschlussmembran (15), die zum Auflegen auf die dem Rahmen abgewandte Seitenfläche des Bodens (3) bestimmt ist.

13. Verfahren zur Kultivierung von Zellen, umfassend die Schritte:
- Auswahl und Bereitstellen eines Mehrkammer-Biochips (2) nach einem der Ansprüche 7 oder 8;
- Einbringen von Medien (18) und Zellen (26, 28, 29, 30, 31) in die vorhandenen Kulturkammern (8, 9, 10).

14. Verfahren nach Anspruch 13, **dadurch gekennzeichnet, dass** in wenigstens eine der Kulturkammern (8, 9, 10) ein Hydrogel eingebracht wird.

15. Verfahren nach Anspruch 13 oder 14, **dadurch gekennzeichnet, dass** in wenigstens einer der Kulturkammern (8, 9, 10) Sphäroide (26) und/oder Organoide (26) erzeugt und/oder kultiviert werden, indem diese in Mikrokavitäten (19) zumindest einer der Trennmembranen (11, 12) angesiedelt werden.
